**Europäisches Patentamt**

**European Patent Office**

**Office européen des brevets**

(11) Veröffentlichungsnummer: **0 100 856**
A1

# EUROPÄISCHE PATENTANMELDUNG

(21) Anmeldenummer: **83106403.5**

(22) Anmeldetag: **01.07.83**

(51) Int. Cl.³: **A 01 N 57/16**
A 01 N 57/14, A 01 N 57/12
//(A01N57/16, 57/12, 57/14, 57/12,
57/12, 55/04, 57/12, 47/34, 57/12,
47/30, 57/12, 47/22, 57/12, 35/10)

(30) Priorität: **10.07.82 DE 3225942**

(43) Veröffentlichungstag der Anmeldung:
**22.02.84 Patentblatt 84/8**

(84) Benannte Vertragsstaaten:
**AT BE CH DE FR GB IT LI LU NL SE**

(71) Anmelder: **A. Nattermann & Cie. GmbH**
**Nattermannallee 1**
**D-5000 Köln 30(DE)**

(72) Erfinder: **Ghyczy, Miklos, Dr.**
**Am Serviesberg 12**
**D-5000 Köln 41(DE)**

(72) Erfinder: **Hager, Jörg-Christian**
**Hermann-Josef-Schmitt-Strasse 48**
**D-5000 Köln 30(DE)**

(72) Erfinder: **Etschenberg, Eugen, Dr.**
**Hirseweg 10**
**D-5000 Köln 41(DE)**

(72) Erfinder: **Wendel, Armin**
**Goethestrasse 20**
**D-5000 Köln 40(DE)**

(74) Vertreter: **Redies, Bernd, Dr. rer. nat.**
**Redies, Redies, Türk & Gille Brucknerstrasse 20**
**D-4000 Düsseldorf 13(DE)**

(54) **Gegen Ektoparasiten wirksame Formulierungen.**

(57) Die vorliegende Erfindung betrifft neue gegen Ektoparasiten wirksame Formulierungen, die aus einem oder mehreren Wirkstoffen und einem oder mehreren Phospholipiden neben üblichen Träger-, Verdünnungs-, Lösungs- und/oder anderen inerten Hilfsmittel bestehen, wobei das Gewichtsverhältnis Wirkstoff bzw. Wirkstoffgemisch zu reinem Phospholipid bzw. Gemisch aus reinen Phospholipiden 1 : 0,5 bis 1 : 5 beträgt. Die Erfindung betrifft weiter Verfahren zur Bekämpfung von Ektoparasiten mit diesen Mitteln und Verfahren zur Herstellung derselben.

EP 0 100 856 A1

Anmelder:    A. Nattermann & Cie GmbH
             Nattermannallee 1, 5000 Köln 30

Titel:       Gegen Ektoparasiten wirksame Formu-
             lierungen

## Beschreibung

Die Erfindung betrifft neue Formulierungen zur Bekämpfung
von Ektoparasiten bei Haus- und Weidetieren. Die neuen Formulierungen weisen eine wesentlich bessere Wirksamkeit auf
als die bisher bekannten Formulierungen.

Zur Bekämpfung von Ektoparasiten, wie Zecken, Fliegen,
Fliegenlarven, Räubemilben, Läusen, Blowflylarven u. a. bei
Haus- und Weidetieren, insbesondere Rindern und Schafen,
werden eine große Anzahl von chemischen Insektiziden mit
mehr oder weniger großem Erfolg eingesetzt.
Nachteil der bisherigen Formulierungen ist zum Beispiel
ihre schlechte Haftung und Verteilung auf den Tierhäuten.
Dadurch muß oft eine hohe Konzentration an Insektiziden
aufgewendet werden. Auch müssen die Maßnahmen oft wiederholt werden. Durch die schlechte Verteilung kommt es auch
vor, daß die Ektoparasiten an schlecht zugänglichen Stellen
nicht ausreichend bekämpft werden können und an anderen
Stellen durch die überhöhte Konzentration eine Schädigung
durch die Insektizide hervorgerufen wird.

Ziel der vorliegenden Erfindung ist es deshalb, neue Formulierungen zur Bekämpfung von Ektoparasiten bereitzustellen,
die eine gute Haftung und eine gute Verteilung der Wirkstoffe

auf der Tierhaut bzw. dem Haarkleid ermöglichen und dadurch die Aufwandmenge an Wirkstoff stark reduziert werden kann.

Es wurde nun überraschenderweise gefunden, daß man hochwirksame Formulierungen zur Bekämpfung von Ektoparasiten bei Haus- und Weidetieren erhalten kann, wenn man die Wirkstoffe zusammen mit einem Phospholipid aus der Gruppe Phosphatidylcholin, den hydrierten Phosphatidylcholinen, Phosphatidylethanolamin, den N-Acyl-phosphatidyl-ethanolaminen, Phosphatidylinosit, Phosphatidylserin, Lysolecithin und Phosphatidylglycerol oder einem Gemisch aus mehreren solcher Phospholipide im Gewichtsverhältnis 1 : 0,5 bis 1 : 5 neben üblichen Träger-, Verdünnungs-, Lösungs-, Versprühungs- und/oder anderen inerten Hilfsmitteln einsetzt.

Durch den Zusatz von Phospholipiden wird eine wesentlich bessere Verteilung und eine bessere Haftung der aktiven Wirkstoffe auf der Tierhaut bzw. dem Haarkleid erzielt und damit auch eine lang anhaltende Wirksamkeit. Dadurch kann die Aufwandmenge an aktivem Wirkstoff bis zu 80 % reduziert werden. Des weiteren ist es möglich, die Behandlungsintervalle wesentlich zu verkürzen. Darüberhinaus zeigen die neuen Formulierungen ein wesentlich besseres Rückstandsverhalten.

Bei den Phospholipiden handelt es sich um meist natürliche, auf jeden Fall ungiftige Produkte, die aufgrund ihrer Natur und Eigenschaften selbst zu keinerlei Umweltbelastung führen.

Zur Herstellung der neuen Formulierungen werden die gegen Ektoparasiten wirksamen Stoffe im Gewichtsverhältnis 1 : 0,5 bis 1 : 5, bevorzugt 1 : 0,5 bis 1 : 3, insbesondere jedoch im Gewichtsverhältnis 1 : 1 bis 1 : 2, mit einem oder mehreren Phospholipiden gemischt.

Als Phospholipide kommen natürliche oder synthetische Phospholipide aus der Gruppe Phosphatidylcholin, den hydrierten Phosphatidylcholinen, Phosphatidylethanolamin, den N-Acyl-

phosphatidylethanolaminen, Phosphatidylinosit, Phosphatidyl-glycerol oder ein Gemisch aus mehreren solcher Phospholipiden, wie z.B. Gemische aus Phosphatidylcholin und Phospha-tidylethanolamin oder Phosphatidylcholin mit Phosphatidyl-ethanolamin und N-Acylphosphatidylethanolamin oder sonstige Phosphatidylcholin-Mischungen mit 20 - 98 % Phosphatidyl-cholin-Anteil in Frage.

Besonders bevorzugt sind natürliche Phosphatidylcholine, die nach den in den folgenden Patentschriften beschriebenen Verfahren erhalten werden können: DE-PS 10 47 579, DE-PS 10 53 299, DE-PS 16 17 679, DE-PS 16 17 680, deutsche Patentanmeldungen P 30 47 048.5, P 30 47 012.3 oder P 30 47 011.2.

Als N-Acyl-phosphatidylethanolamine kommen insbesondere diejenigen in Betracht, in denen die Acylgruppe sich von gesättigten oder olefinisch ungesättigen Fettsäuren mit 2 - 20 Kohlenstoffatomen oder die gesättigten oder einmal olefinisch ungesättigten mit 14, 16, 18 oder 20 Kohlenstoffatomen in Frage.

Als aktive Wirkstoffe gegen Ektoparasiten kommen alle Insektizide,die gegen Zecken, Fliegen, Dassellarven, Räubemilben, Läuse, Blowflylarven und andere Ektoparasiten wirksam sind. Als Beispiele seien Phosphate, Carbamate und Amidine genannt.

Phosphorverbindungen, wie z.B.
O-(4-Brom-2,5-dichlor-phenyl)-O,O-diethyl-monothiophosphat (Bromophosethyl),
S-(p-Chlorphenylthiomethyl)-O,O-diethyl-dithiophosphat (Carbophenothion),
Diethyl-[2-chlor-1-(2,4-dichlorphenyl)]-vinyl-phosphat (Chlorfenvinphos),
(2,2,2-trichlor-1-hydroxyethyl)-phosphat (Chlorophos),
O,O-Diethyl-O-(3,5,6-trichlor-2-pyridyl)-monothiophosphat (Chlorpyrifos),
O-3-Chloro-4-methylcoumarin-7-yl-O,O-diethylphosphorothioat (Coumaphos),
4-tert.-butyl-2-Chlorphenyl-methyl-phosphoramidat (Crumofat),
O-(2,2-Dichlorvinyl)-O,O-dimethylphosphat (Dichlorvos),

S,S'-(1,4-Dioxan-2,3-diyl)-0,0,0',0'-tetraethyl-di-(phosphadithioat (Dioxathion),
0,0-Diethyl-0-(2-isopropyl-6-methylpyrimidin-4-yl)-monothiophosphat (Diazinon),
0,0-Dimethyl-N-dimethylcarbamoyl-1-methyl-vinyl-phosphat (Ektaphos),
S,S'-Methylen-bis-(0,0-diethyldithiophosphat) (Ethion),
7-Chlor-bicyclo-(3,2,0)-hepta-2,6-dien-6-yl-dimethylphosphat (Heptenophos),
S-/1,2-bis-(Ethoxy-carbonyl)-ethyl/-0,0-diemthyl-dithiophosphat (Malathion),
3-(0,0-Diethyl-dithiophosphoryl-methyl)-6-chlor-benzoxazolon (Phosalon),
0,0-Dimethyl-S-phthalimidomethyl-dithiophosphat (Phosmet).

Carbamate, wie z.B.:
N-Methyl-1-naphthyl-carbamat (Carbaryl),
2-Isopropoxyphenyl-N-methylcarbamat (Propexur),
N-Methyl-(3,5-di-tert.-butyl)-phenyl-carbamat (Butacarb),
N-(4-Chlorphenyl)-aminocarbonyl-2,6-difluorbenzamid (Diflubenzuron).

Sonstige, gegen Ektoparasiten wirksame Substanzen:
N'-(4-Chlor-2-methylphenyl)-N,N-dimethyl-formamidin (Chlordimeform),
N-Methyl-bis-(2,4-xylyliminomethyl)-amin (Amitraz),
3-(4-Chlor-o-tolyl)-1,1-dimethyl-thioharnstoff (Chlormethiuron),
Tricyclohexyl-zinn-hydroxid (Cyhexatin).

Zur Herstellung der neuen gegen Ektoparasiten wirksamen Formulierungen wird das entsprechende Phospholipid in einem geeigneten Lösungsmittel, wie z.B. Toluol, Essigester, Xylol, Benzin, Methanol oder Ethanol oder Gemische dieser Lösungsmittel gelöst. Die Auswahl des Lösungsmittel richtet sich nach den Lösungseigenschaften des eingesetzten Wirkstoffes. In die Phospholipid-Lösung wird ggfs. unter Erwärmung der Wirkstoff oder eine im Handel erhältliche Formulierung aufgelöst. Nach Beendigung des Lösungsvorganges wird das Lösungsmittel im Vakuum unter Erwärmung abdestilliert. Das so erhaltene Produkt wird ggfs. durch Zugabe geeigneter inerter Hilfsmittel wie Füllstoffe, Quellmittel, Emulgatoren, Netzmittel, Versprühungsmittel zu üblichen handelsfähigen Produkten verarbeitet.

Die neuen gegen Ektoparasiten wirksamen Formulierungen können auch hergestellt werden, indem der Wirkstoff oder eine handelsübliche Formulierung, das Phospholipid und die noch erwünschten Hilfsmittel zusammen im Lösungsmittel oder Lösungsmittelgemisch durch Erwärmen gelöst bzw. aufgeschlämmt werden und dann das oder die Lösungsmittel im Vakuum abgezogen werden. Erhalten wird eine fertige Mischung, die sofort zur Anwendung bereit steht.

Bei Wirkstoffen, die in Wasser oder Wasser-Alkohol-Mischungen löslich sind, wird vorteilhafterweise der Wirkstoff zuerst in Wasser oder Wasser-Alkohol-Mischungen gelöst und das Phospholipid oder die Phospholipidmischung unter Rühren oder durch die Anwendung von Ultraschall zu einer Lösung oder Emulsion verarbeitet. Die gegebenenfalls benötigten Emulgatoren können vor oder nach diesem Rührvorgang zugesetzt werden. Die so erhaltene Emulsion oder Lösung wird üblicherweise, wie z.B. durch Destillation, Sprühtrocknen, Lyophilisieren, von dem Lösungsmittelgemisch oder Wasser befreit. Das resultierende Produkt kann als Trockenprodukt ggfs. unter Zusatz von Hilfs- und Füllstoffen eingesetzt werden. Auch kann das Produkt wieder unter Zusatz von geeigneten Hilfsstoffen in Wasser emulgiert oder gelöst werden und als flüssiges Produkt eingesetzt werden. Trägerstoffe sind z.B. Talkum, Kaolin, Bentonit, Kieselgur, Kalk oder Gesteinsmehl. Weitere Hilfsmittel sind z.B. oberflächenaktive Verbindungen, wie Seifen (Fettsäuresalze), Fettalkoholsulfonate oder Alkylsulfonate. Als Stabilisatoren oder Schutzkoloide können Gelatine, Kasein, Albumin, Stärke oder Methylcellulose eingesetzt werden. Desweiteren können auch Spreitmittel wie Silikonöle, Fettsäureester, Triglyceride, Fettalkohol oder Fettsäuren eingesetzt werden.

Die neuen gegen Ektoparasiten wirksamen Formulierungen können in flüssiger oder fester Form eingesetzt werden, z.B. als Stäubemittel, Spray oder als Lösung, Emulsion oder Suspension für Dip-oder Pour-on-Formulierungen.

Die neuen Formulierungen können z.B. bei Zecken, Fliegen, Dassellarven, Räudemilfem, Läusen, Blowfly-Larven bei Haustieren, wie Hunde und Katzen, sowie Weidetieren wie Rinder, Schafe,Ziegen, Pferde, eingesetzt werden.

Beispiel 1

1 Liter Emulsionskonzentrat (250 g/l) mit N'-(4-Chlor-2-methylphenyl)-N,N-dimethylformamidin als aktivem Wirkstoff wird hergestellt.

250g   N'-(4-Chlor-2-methylphenyl)-N,N-dimethylformamidin
250g   Phospholipid in 100 ml Ethanol
 10g   Xylol

werden mit Shellsol N.auf 1 Liter aufgefüllt und gelöst.

1 Liter dieses Emulsionskonzentrates mit 250 g/l ist genauso wirksam wie 1 Liter eines üblichen Handelsproduktes mit 500 g/l Gehalt an Wirkstoff.

Beispiel 2

150 ml eines Emulsionskonzentrates wird hergestellt.
40 g   Bromophosethyl
40 g   Phospholipid
40 ml  Isophoron
15 ml  Glycerin
 5 ml  Cremaphor EL
 5 ml  Marlowet  IHV

werden zusammengegeben und mit einem Xylolgemisch auf 150ml aufgefüllt

Beispiel 3

Herstellung von 100 g eines 1 %igen Puders:
86 g     Talk, feinstgepulvert DAB 7
10,9 g   Bentonit Typ APV, Fa. Mainland
 1,1 g   Titandioxyd, Bayertitan A

werden homogen gemischt. Auf diese Pulvermischung wird eine methanolische Lösung von

1 g Phospholipid
1 g Chlorpyrifos

aufgesprüht. Das Produkt wird getrocknet.

Beispiel 4

150 ml eines 33 %igen Emulsionskonzentrates werden folgendermaßen hergestellt:

50 g  Phospholipid, gelöst in 15 ml Ethanol wird mit

50 g Dichlorvos

5 g Marlowet IHF

zusammengegeben und mit Isopropanol auf 150 ml aufgefüllt.


Beispiel 5

50 g eines 5 %igem Pulvers werden hergestellt.

Die Teile

79 g  Talk, feinstgepulvert, DAB 7

10 g  Bentonit, Typ APV, Fa. Mainland

1 g  Titandioxyd, Bayertitan, Fa. Bayer

werden homogen vermischt. Auf die Pulvermischung wird eine methanolische Lösung von 5 g Coumaphos und Phospholipid aufgesprüht und das Produkt getrocknet.


Beispiel 6

Ein  16 %iges Emulsionskonzentrat wird hergestellt.

Die Bestandteile

16 g  Heptenophos

16 g  Phospholipid

16 g  Isophoron

4 g  Softigen

2 g  Marlowet IHF

werden mit eine Xylolmischung auf 100 ml aufgefüllt.


Beispiel 7

100 g eines 2 %igen Staubes werden hergestellt.

81 g  Talk, feinstgepulvert, DAB 7

11 g  Bentonit, Typ APV, Fa. Mainland

1 g  Titandioxyd, Bayertitan, Fa. Bayer

werden gemischt und die methanolische Lösung von 2 g Carbaryl und 5 g Phospholipid aufgesprüht. Das Produkt wird getrocknet.

Beispiel 8

100 ml eines 16 %igen Emulsionskonzentrates werden folgendermaßen hergestellt:

20 g Isophoron

15 g Xylol

15 g Toluol

9 g Cremophor EL

werden zusammengegeben und erwärmt. Nacheinander werden in dieser Mischung aufgelöst:

16 g Phospholipid

16 g Propoxur

9 g N-(2-Hydroxyethyl)-capronsäureamid

Beispiel 9

100 g eines Suspensionskonzentrates (6 %) werden aus den Bestandteilen

6 g Diflubenzuron (fein gemahlen)

24 g Phospholipid

42 g Isophoron

21 g Glycerin

7 g Cremophor E

mit Hilfe eines Ultraturax hergestellt. Der Mischvorgang dauert 10 Minuten.

Die in den vorstehenden Beispielen 1 bis 9 verwendeten
Phospholipid-Produkte hatten die folgende Zusammensetzung,
wobei die angegebenen Prozente Gewichtsprozente sind:

Beispiel 1:
96 % Phosphatidylcholin
 4 % sonstige Phospholipide

Beispiel 2:
25 % Phosphatidylcholin
25 % Phosphatidylethanolamin
2o % Phosphatidylinositol
3o % sonstige Phospholipide

Beispiel 3:
55 % Phosphatidylcholin
25 % Phosphatidylethanolamin
 2 % Phosphatidylinositol
18 % sonstige Phospholipide

Beispiel 4:
38 % Phosphatidylcholin
16 % N-acylphosphatidylethanolamin
24 % Phosphatidylethanolamin
3o % sonstige Phospholipide

Beispiel 5:
4o % Phosphatidylcholin
3o % Phosphatidylethanolamin
25 % N-acylphosphatidylethanolamin
 5 % sonstige Phospholipide

Beispiel 6:
42 % Phosphatidylcholin
25 % Phosphatidylethanolamin
25 % N-acylphosphatidylethanolamin
 8 % sonstige Phospholipide

0100856

Beispiel 7:

45 % Phosphatidylcholin

25 % Phosphatidylethanolamin

21 % N-acylphosphatidylethanolamin

9 % sonstige Phospholipide

Beispiel 8:

4o % Phosphatidylcholin

28 % Phosphatidylethanolamin

27 % N-acylphosphatidylethanolamin

5 % sonstige Phospholipide

Beispiel 9:

4o % Phosphatidylcholin

28 % Phosphatidylethanolamin

27 % N-acylphosphatidylethanolamin

5 % sonstige Phospholipide

Patentansprüche

1. Formulierungen zur Bekämpfung von Ektoparasiten, dadurch gekennzeichnet, daß sie aus
   a) einem oder mehreren gegen Ektoparasiten wirksame Stoffe und

   b) einem oder mehreren Phospholipiden aus der Gruppe Phosphatidylcholin, den hydrierten Phosphatidylcholinen, Phosphatidylethanolamin, den N-Acyl-phosphatidylethanolaminen, Phosphatidylinosit, Phosphatidylserin, Lysolecithin und Phosphatidylglycerol
   
   im Gewichtsverhältnis a : b von 1 : 0,5 bis 1 : 5 neben üblichen Träger-, Verdünnungs-, Lösungs-, Versprühungs- und/oder anderen inerten Hilfsmittel bestehen.

2. Formulierungen gemäß Anspruch 1, dadurch gekennzeichnet, daß die Bestandteile a) und b) im Gewichtsverhältnis 1 : 0,5 bis 1 : 3 vorliegen.

3. Formulierungen gemäß Anspruch 1 oder 2, dadurch gekennzeichnet, daß die Bestandteile a) und b) im Gewichtsverhältnis 1 : 1 bis 1 : 2 vorliegen.

4. Formulierungen gemäß Anspruch 1 bis 3, dadurch gekennzeichnet, daß als Phospholipid Phospholipid, Phosphatidylcholin und Phosphatidylethanolamin oder Mischungen aus Phosphatidylcholin, Phosphatidylethanolamin und N-Acyl-phosphatidylethanolamin eingesetzt werden.

5. Formulierungen gemäß Ansprüchen 1 bis 4, dadurch gekennzeichnet, daß als Phospholipid ein Phospholipid mit einem Gehalt von 20 - 98 % Phosphatidylcholin eingesetzt wird.

6. Formulierungen gemäß Ansprüchen 1 bis 5, dadurch gekennzeichnet, daß als gegen Ektoparasiten wirksamer Stoff Insektizide aus der Gruppe der Phosphorverbindungen, Carbamate, Amidine oder Thioharnstoffe eingesetzt werden.

7. Formulierung gemäß Anspruch 6, dadurch gekennzeichnet, daß als gegen Ektoparasiten wirksame Stoffe Bromophosethyl, Carbophenothion, Chlorfenuinphos, Chlorophos, Chlorpyrifos, Coumaphos, Crufomat, Dichlorvos, Dioxathion, Diazinon, Ektaphos, Ethion, Hetenaphos, Malathion, Phosalon, Phosmet, Carbaryl, Propoxur, Butacarb, Diflubenzuron, Chlordimeform, Amitraz, Chloromethiuron und/oder Cyhexatin eingesetzt werden.

8. Verwendung der Formulierungen gemäß einem oder mehreren der Ansprüche 1 - 7 zur Bekämpfung von Ektoparasiten.

9. Verfahren zur Herstellung von Formulierungen gemäß einem oder mehreren der Ansprüche 1 - 8, dadurch gekennzeichnet, daß das Phospholipid in einem organischen Lösungsmittel bzw. Lösungsmittelgemisch gelöst wird und in diese Lösung ggfs. durch Erwärmen und/oder Rühren der Wirkstoff gelöst und anschließend das Lösungsmittel oder Lösungsmittelgemisch im Vakuum abgezogen wird und die erhaltene Mischung unter Zusatz üblicher Füll- und Hilfsmittel

in eine übliche Anwendungsform überführt wird.

10. Verfahren zur Herstellung von Formulierungen gemäß einem oder mehreren der Ansprüche 1 - 5, dadurch gekennzeichnet, daß das oder die Wirkstoffe mit einem oder mehreren Phospholipiden ggfs. unter Zusatz üblicher Füll- und Hilfsmittel in einem organischen Lösungsmittel gelöst bzw. aufgeschlämmt und das Lösungsmittel danach abgezogen wird.

11. Verfahren zur Herstellung von Formulierungen gemäß einem oder mehreren der Ansprüche 1 - 5, dadurch gekennzeichnet, daß ein oder mehrere, in Wasser oder Alkohol lösliche Wirkstoffe in Wasser und Wasser-Alkohol-Mischungen zusammen mit einem oder mehreren Phospholipiden unter Rühren und/oder leichter Erwärmung und/oder Ultraschall gelöst und das Lösungsmittel bzw. Lösungsmittelgemisch abgezogen wird.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| P,X | EP-A-0 068 297 (A. NATTERMANN & CIE.) <br> * Seite 3, Zeilen 1-18; Seite 9, Zeilen 27-30; Seite 10, Zeilen 9,18,19,31,32; Seite 11, Zeilen 1,2,5,6,14,17,18; Seite 12, Zeilen 1,2; Seite 13, Zeilen 21,34; Seite 14, Zeilen 11-13; Ansprüche 1-4, 15-17, 19-22, 24-27 * <br><br> --- | 1-11 | A 01 N   57/16 <br> A 01 N   57/14 <br> A 01 N   57/12  // <br> (A 01 N   57/16 <br> A 01 N   57/12 <br> A 01 N   57/14 <br> A 01 N   57/12 <br> A 01 N   57/12 <br> A 01 N   55/04 <br> A 01 N   47/34 <br> A 01 N   47/30 <br> A 01 N   47/22 <br> A 01 N   35/10  ) |
| A | US-A-4 241 046 (D.P. PAPAHADJOPOULOS et al.) <br> * Spalte 3, Zeile 65 - Spalte 4, Zeile 16; Spalte 4, Zeile 39 - Spalte 6, Zeile 16; Anspruch * <br><br> ----- | 1,4,5, 9-11 | |

| | | | RECHERCHIERTE SACHGEBIETE (Int. Cl. ³) |
|---|---|---|---|
| | | | A 01 N |

Der vorliegende Recherchenbericht wurde für alle Patentansprüche erstellt.

| Recherchenort <br> DEN HAAG | Abschlußdatum der Recherche <br> 21-10-1983 | Prüfer <br> FLETCHER A.S. |
|---|---|---|

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82